(19) Europäisches Patentamt
European Patent Office
Office européen des brevets

(11) EP 1 964 530 A1

(12) EUROPEAN PATENT APPLICATION

(43) Date of publication:
**03.09.2008 Bulletin 2008/36**

(21) Application number: **08003367.3**

(22) Date of filing: **25.02.2008**

(51) Int Cl.:
***A61B 17/22*** *(2006.01)* ***A61B 17/32*** *(2006.01)*
***A61B 18/12*** *(2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **28.02.2007 US 679986**

(71) Applicant: **Olympus Medical Systems Corp.**
**Tokyo 151-0072 (JP)**

(72) Inventor: **Kimura, Kenichi**
**Hachioji-shi**
**Tokyo 192-8512 (JP)**

(74) Representative: **von Hellfeld, Axel**
**Wuesthoff & Wuesthoff**
**Patent- und Rechtsanwälte**
**Schweigerstrasse 2**
**81541 München (DE)**

# (54) Treatment apparatus for operation

(57) A treatment apparatus (1) for operation is configured to operate in a plurality of output modes. A hand piece (4) is provided with a probe configured to supply a high-frequency current, and an ultrasonic transducer which is connected to the probe and activates ultrasonically the probe. A high frequency driving circuit (3) supplies a high-frequency current to the probe. An ultrasonic transducer driving circuit (2) drives the ultrasonic transducer. An operation switch (31) selects a first output mode and a second output mode. A controller controls a high-frequency output from the high frequency driving circuit (3) and an ultrasonic output from the ultrasonic transducer driving circuit (2), to operate the treatment apparatus (1) for operation in an output mode selected by the operation switch (31).

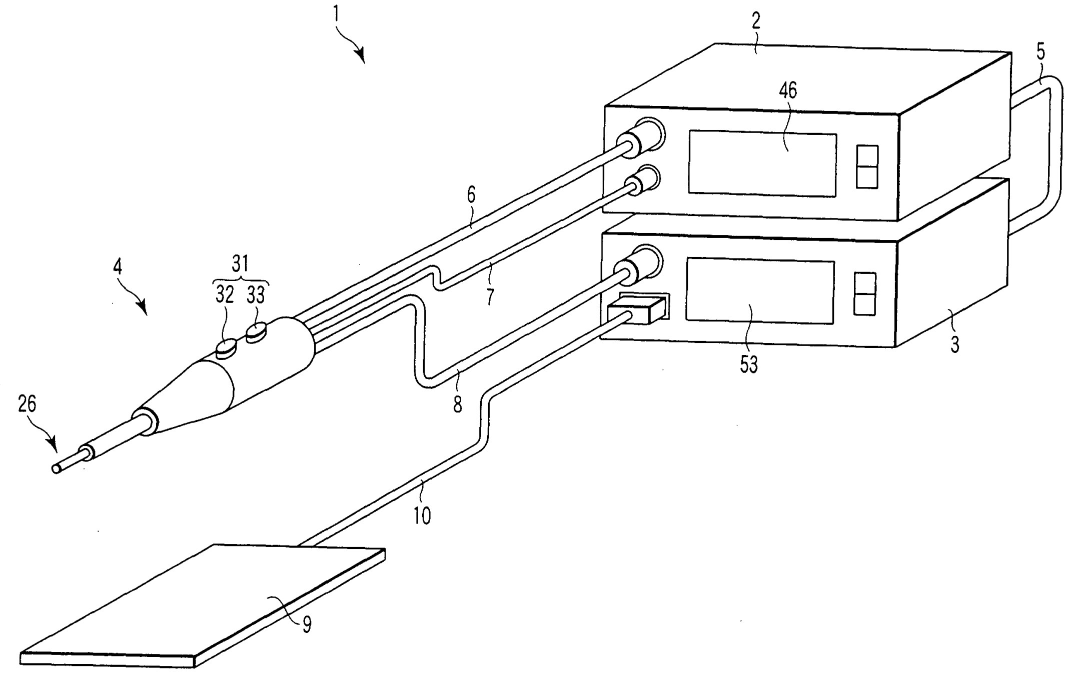


FIG. 1



Printed by Jouve, 75001 PARIS (FR)

## Description

The present invention relates to a treatment apparatus for operation.

A treatment apparatus for operation has been known. For example, Jpn. Pat. Appln. KOKAI Publication No. 2002-306507 discloses an apparatus for operation 1 having a scalpel tip electrode 26 forming an electrode unit for treating a living tissue by using a high-frequency current, a high-frequency current supply unit 50 for supplying a high-frequency current to the scalpel tip electrode 26, and an ultrasonic activation supply unit 10 for supplying ultrasonic activation to the scalpel tip electrode 26. The treatment apparatus for operation 1 is configured so that the scalpel tip electrode 26 can obtain ultrasonic activation while being supplied with a high-frequency current to prevent burning and sticking of a living tissue to the scalpel tip electrode 26.

A hand piece 40 is provided with a hand switch 43. The hand switch 43 has electrical output switches 44a and 44b for turning on/off the output from the high-frequency current supply unit 50, and an ultrasonic output switch 45 for turning on/off the output from the ultrasonic activation supply unit 10. Each of these switches 44a, 44b and 45 is configured to control turning on/off of each output mode.

According to a first aspect of the invention, there is provided a treatment apparatus for operation operable in a plurality of output modes comprising:

a hand piece provided with a probe capable of supplying a high-frequency current, and an ultrasonic transducer connected with the probe for ultrasonically activating the probe;
a high frequency driving circuit for supplying a high-frequency current to the probe;
an ultrasonic transducer driving circuit for driving the ultrasonic transducer;
an operation switch for selecting a first output mode and a second output mode; and
a controller which controls a high-frequency output from the high frequency driving circuit and an ultrasonic output from the ultrasonic transducer driving circuit, for operating the treatment apparatus for operation in an output mode selected by the operation switch.

According to a second aspect of the invention, there is provided a treatment apparatus for operation according to the first aspect, wherein the operation switch is composed of a two-step operation switch configured to be set to a first operation position and a second operation position.

According to a third aspect of the invention, there is provided a treatment apparatus for operation according to the second aspect, wherein the controller operates the treatment apparatus for operation in the first output mode when the operation switch is set to the first operation position, and operates the treatment apparatus for operation in the second output mode when the operation switch is set to the second operation position.

According to a fourth aspect of the invention, there is provided a treatment apparatus for operation according to the third aspect, wherein the first output mode is a high-frequency output mode to output a high-frequency signal from the high frequency driving circuit, or an ultrasonic output mode to output an ultrasonic signal from the ultrasonic transducer driving circuit; and the second output mode is an output mode combining the high-frequency output mode and the ultrasonic output mode.

According to a fifth aspect of the invention, there is provided a treatment apparatus for operation according to the first aspect, further comprising a fluid supply unit for supplying fluid to the hand piece, and a suction unit for collecting fluid from the hand piece.

According to a sixth aspect of the invention, there is provided a treatment apparatus for operation according to the fifth aspect, wherein the first and second output modes include a fluid supply/suction output mode to control a fluid supply output of the fluid supply unit and/or a suction output of the suction unit; and the operation switch includes an operation switch for setting the fluid supply/suction output mode.

According to a seventh aspect of the invention, there is provided a hand piece used in a treatment apparatus for operation operable in a plurality of output modes, the hand piece comprising a probe capable of supplying a high-frequency current; an ultrasonic transducer which is connected to the probe, and ultrasonically activates the probe; and an operation switch for selecting a first output mode and a second output mode, wherein a high-frequency output from a high frequency driving circuit for supplying a high-frequency current to the probe, and an ultrasonic output from an ultrasonic transducer driving circuit for driving the ultrasonic transducer are controlled so as to operate the treatment apparatus for operation in an output mode selected by the operation switch.

The invention can be more fully understood from the following detailed description when taken in conjunction with the accompanying drawings, in which:

FIG. 1 is a diagram showing a configuration of a treatment apparatus for operation 1 according to a first embodiment of the invention;
FIG. 2 is a sectional view of a hand piece 4;
FIG. 3 is an enlarged view of a treatment unit 26;
FIG. 4 is an enlarged view of a treatment unit 26;
FIG. 5 is a block diagram showing the configuration of an ultrasonic driving unit 2 and a high frequency driving unit 3;
FIG. 6 is a table showing a list of output modes of the treatment apparatus for operation 1;
FIG. 7 is a graph explaining the output characteristics of high-frequency output;
FIG. 8 is a graph explaining the output characteristics of high-frequency output;

FIG. 9 is a graph explaining the output characteristics of ultrasonic output;

FIG. 10 is a graph explaining the output characteristics of ultrasonic output;

FIG. 11 is a table showing an example of output modes set in the treatment apparatus for operation 1;

FIG. 12 is a table showing an example of output modes set in the treatment apparatus for operation 1;

FIGS. 13A and 13B are diagrams explaining the configuration and operation of a hand switch 31;

FIGS. 14A and 14B are diagrams explaining the configuration and operation of a hand switch 31;

FIGS. 15A and 15B are diagrams explaining the configuration and operation of a hand switch 31;

FIG. 16 is a graph explaining the output characteristics of high-frequency output and ultrasonic output;

FIG. 17 is a front view of a hand piece 55;

FIG. 18 is a graph showing a list of output modes of a treatment apparatus for operation;

FIG. 19 is a graph showing an example of output modes set in a treatment apparatus for operation;

FIG. 20 is a diagram showing the configuration of a treatment apparatus for operation 71;

FIG. 21 is a table showing a list of output modes of the treatment apparatus for operation 71; and

FIG. 22 is a table showing an example of output modes set in the treatment apparatus for operation 71.

Embodiments of the present invention will be explained in detail hereinafter with reference to the accompanying drawings.

(Embodiment 1)

(Configuration)

FIG. 1 shows a configuration of a treatment apparatus for operation 1 according to a first embodiment of the invention. As shown in FIG. 1, the apparatus for operation 1 comprises an ultrasonic driving unit 2, a high frequency driving unit 3, and a hand piece 4. The ultrasonic driving unit 2 and high frequency driving unit 3 are connected by a communication cable 5. The hand piece 4 is connected to the ultrasonic driving unit 2 by an output connection cable 6 and a switch (SW) connection cable 7. The hand piece 4 is connected to the high frequency driving unit 3 by an output connection cable 8. A patient plate 9 is connected to the high frequency driving unit 3 by a connection cable 10.

FIG. 2 shows a sectional view of the hand piece 4. As shown in FIG. 2, the hand piece 4 is provided with a substantially cylindrical case 11 in the rear end side. The case 11 is made of insulating material such as plastic. An ultrasonic transducer 12 (bolt-fixed Langevin type transducer) for generating ultrasonic activation is fixed inside the case 11. The ultrasonic transducer 12 is provided with a plurality of ring-shaped piezoelectric elements 13 (6 in this embodiment) for converting electric power supplied from the ultrasonic driving unit 2 into ultrasonic activation. In the distal end side of the piezoelectric element 13, a horn 14 for amplifying the ultrasonic activation generated by the piezoelectric element 13 is provided. The horn 14 is made of metallic material such as titanium, duralumin, and stainless steel. A bolt unit 15 is provided in the rear end side of the horn 14. The bolt unit 15 is inserted into the piezoelectric element 13 through a pipe-shaped insulating member 16. A metallic nut 17 is provided in the rear end side of the piezoelectric element 13. The piezoelectric element 13 is fastened by the nut 17 and the bolt unit 15 of the horn 14 through ring-shaped insulating members 18 and 19. Therefore, the piezoelectric element 13 is electrically insulated from the horn 14 and nut 17. A conductor 22 in the output connection cable 6 is connected to the piezoelectric element 13 through metallic bridges 20 and 21, whereby electric power for ultrasonic driving is supplied to the piezoelectric element 13. A conductor 23 in the output connection cable 8 is connected to the nut 17, whereby a high-frequency current is supplied to the nut and horn 14. The output connection cables 6 and 8 are extended from the rear end side of the case 11. In the rear end side of the case 11, an elastic member 24 for protecting the output connection cables 6 and 8 is provided as one unit with the case 11.

In the distal end side of the horn 14, a probe 25 is provided for transmitting ultrasonic activation amplified by the horn 14. The horn 14 and probe 25 are fastened with a screw. Like the horn 14, the probe 25 is made of metallic material such as titanium, duralumin, and stainless steel. In the distal end side of the probe 25, a treatment unit 26 for treating a living tissue is provided. The treatment unit 26 shown in FIG. 2 is a substantially flat plate like a spatula. The treatment unit may be hook-shaped as in FIG. 3 or ball-shaped as in FIG. 4.

In the distal end side of the case 11, a sheath main body 27 is provided to insert the probe 25. The sheath main body 27 is made of insulating material such as plastic. In the distal end side of the sheath main body 27, a metal pipe 28 and an insulating tube 29 covering the metal pipe 28 are provided. In the distal end side of the metal pipe 28, a support member 30 is provided to prevent contact between the probe 25 and metal pipe 28. The insulating tube 29 and support member 30 are made of resin material such as PTFE with good electrical insulation. In the sheath main body 27, a first switch 32 and a second switch 33 are provided as a hand switch (operation switch) 31. The first and second switches 32 and 33 are provided with an electrical circuit board 34. The electrical circuit board 34 is housed in the sheath main body 27. The electrical circuit board 34 is connected to a conductor 35 in the switch connection cable 7. The switch connection cable 7 is extended from the rear end of the sheath main body 27. Therefore, electric signals (open, shorted) of the first and second switches 32 and 33 are transmitted to the ultrasonic driving unit 2.

FIG. 5 is a block diagram showing the configuration of the ultrasonic driving unit 2 and high frequency driving unit 3. Inside the ultrasonic driving unit 2, an ultrasonic transducer driving circuit 37 is provided. Here, PLL control system is used as a resonance tracking system, and a current control system is used as an amplification control system. The ultrasonic transducer driving circuit 37 is provided with an output circuit, in which a phase tracking circuit (PLL) 38, a voltage control amplifier (VCA) 39 as a multiplier, a power amplifier (AMP) 40 for generating a current to give power to the ultrasonic transducer 12, a voltage/current detector (DET) 41 and an output transformer 42 are sequentially connected in series. The hand piece 4 is connected to the output port of the output transformer 42 through the output connection cable 6. The ultrasonic transducer driving circuit 37 and hand piece 4 are electrically isolated by the output transformer 42. The phase tracking circuit (PLL) 38 is a circuit for tracking the resonance frequency of the ultrasonic transducer 12, and driving a resonance point. The phase tracking circuit (PLL) 38 is connected with the voltage/current detector (DET) 41. The voltage/current detector (DET) 41 includes a circuit for detecting a phase signal of voltage and current for PLL control, or detecting the largeness of current flowing in the ultrasonic transducer 12. The ultrasonic transducer driving circuit 37 is provided with a differential amplifier 43, a D/A converter 44, and a CPU 45. The CPU 45 is connected to the D/A converter 44 and an operation display panel 46. The CPU 45 is connected to the first and second switches 32 and 33 through the switch connecting cable 7. The voltage/current detector (DET) 41 is connected to one input terminal of the differential amplifier 43, and the D/A converter 44 is connected to the other input terminal. The D/A converter 44 is configured to generate a signal to instruct the largeness of current corresponding to the ultrasonic output condition instructed by the CPU 45. The output end of the differential amplifier 43 is connected to the voltage control amplifier (VCA) 39. The differential amplifier 43 compares the signal generated by the D/A converter 44 with the largeness of the current detected by the voltage/current detector (DET) 41, amplifies the signal and current to the same largeness, and supplies the amplified output to the voltage control amplifier (VCA) 39. Therefore, the largeness of the voltage applied to the ultrasonic transducer 12 is controlled, so that the current flowing in the ultrasonic transducer 12 becomes the same as the output condition instructed by the CPU 45.

A high frequency driving circuit 48 is provided in the high frequency driving unit 3. The high frequency driving circuit 48 is provided with an output circuit, in which a variable voltage source (switch power supply) 49 for supplying power for high-frequency output and control, a power amplifier (AMP) 50 and a sensor 51 are sequentially connected in series. The sensor 51 is connected to the hand piece 4 through the output connection cable 8, and the patient plate 9 through the connection cable 10. The high frequency driving circuit 48 is provided with an output controller 52. The output controller 52 is connected with the sensor 51, variable voltage source (SW power supply) 49, power amplifier (AMP) 50 and operation display panel 53. The variable voltage source (SW power supply) 49 supplies power for high-frequency output and control. The power amplifier (AMP) 50 amplifies a high-frequency power, and shapes an output waveform. The sensor 51 monitors a high-frequency output (voltage/current values), and sends a monitor signal to the output controller 52. The output controller 52 sends a control signal to the variable voltage source (SW power supply) 49 and power amplifier (AMP) 50, based on the monitor signal from the sensor 51. The high-frequency output is control in this way.

The CPU 45 of the ultrasonic transducer driving circuit 37 is connected to the output controller 52 of the high frequency driving circuit 48 through the communication cable 5 capable of transmitting a signal in two ways. The CPU 45 sends a control signal of the hand switch 31 to the output controller 52. The output controller 52 gives the CPU 45 a signal indicating the largeness of high frequency impedance (impedance between the hand piece 4 and patient plate 9) calculated based on the monitor signal (voltage/current values) of the sensor 51. The operation display panel 46 of the ultrasonic driving unit 2 is connected to the operation display panel 53 of the high frequency driving unit 3 through the CPU 45, communication cable 5 and output controller 52. Settings and display contents are interlocked between the operation display panels 46 and 53.

FIG. 6 shows a list of output modes of the treatment apparatus 1 for operation. Like a conventional monopolar electric surgical knife, the high frequency driving unit 3 enables high-frequency output in various modes: Cut-Pure (cut output including almost no hemostatic component), Cut-Blend (cut output including a certain hemostatic component), (Coag-Soft (coagulation output with weak exfoliation component), Coag-Hard (coagulation output with strong exfoliation component), and Coag-Spray (coagulation output used for uniform hemostasis by discharging in a wide area of tissue). The ultrasonic driving unit 2 enables ultrasonic output in output modes: On (ultrasonic output keeping a set value of amplitude of ultrasonic activation), and Auto (ultrasonic output changing amplitude of ultrasonic activation according to impedance in the high frequency output).

In the treatment apparatus for operation 1, the high-frequency output mode and ultrasonic output mode can be combined. It is also possible to combine Off of the high-frequency output (no output) and On of the ultrasonic output (ultrasonic output keeping a set value of amplitude of ultrasonic activation) or Max of the ultrasonic output (ultrasonic output keeping a maximum value of amplitude of ultrasonic activation) (only the ultrasonic output is obtained). It is also possible to combine the output modes of high-frequency output (e.g. Cut-Pure and Coag-Hard) and Off of the ultrasonic output mode (no output) (only the high-frequency output is obtained).

FIG. 7 and FIG. 8 show examples of the output characteristics (load characteristics) of the high-frequency output. FIG. 7 is a graph showing the impedance in the high-frequency output (the impedance between the hand piece 4 and patient plate 9) on the horizontal axis, and the high-frequency output when the maximum output is set on the vertical axis. The curve a indicates the output characteristic in Coag-Soft, and the curve b indicates the output characteristic in Coag-Hard. Like a conventional monopolar electric surgical knife, when the impedance is increased, the high-frequency output is decreased in both Coag-Soft and Coag-Hard. The high-frequency output when the impedance is increased is slower in Coag-Hard than Coag-Soft. This output characteristic difference acts upon the strength of exfoliation component.

The curve c (thick line) in FIG. 8 indicates the output characteristic when the output is set to 50W in Coag-Hard. As indicated by the curve c, the high-frequency output is kept at 50W until the impedance is increased up to approximately 2.5 kΩ, and decreased after the impedance is increased to higher than 2.5 kΩ. The high-frequency output becomes approximately 25W at the impedance of 5 kΩ.

FIG. 9 and FIG. 10 show the output characteristics of a high-frequency output. In FIG. 9 and FIG. 10, the horizontal axis represents the impedance in the high-frequency output (impedance between the hand piece 4 and patient plate 9), and the vertical axis represents the ultrasonic output. The ultrasonic output is maximum at 100% (maximum amplitude of ultrasonic activation). The activation velocity of the hand piece 4 in the treatment unit 26 is desirably in a range of 7.4m/s - 22.1m/s at the setting of 100%. The activation velocity V is expressed by the following equation.

$$V = \pi \cdot x \cdot fr$$

Where, x is an amplitude (peak-to-peak value) of ultrasonic activation, and fr is a resonance frequency of ultrasonic activation. Namely, at the setting of 100%, the activation velocity is preferably in a range of 100 μm - 300 μm when fr is 23.5 kHz, and 50 μm - 150 μm when fr is 47 kHz.

The line d in FIG. 9 indicates the output characteristic when the output is set to 70% in the ultrasonic output mode On, and the line e indicates the output characteristic in the ultrasonic output mode Max. In both output characteristics, a certain ultrasonic output (amplitude of ultrasonic activation) is kept irrespectively of the largeness of impedance.

The line f indicates an example of output characteristic of the ultrasonic output mode Auto. As shown in this graph, when the impedance is increased, the ultrasonic output is increased stepwise. The ultrasonic output is kept at 30% until the impedance is increased to 2 kΩ, 70% in a range of 2 kQ - 4 kΩ, and 100% after the impedance is increased to 4 kΩ.

The output mode corresponding to the hand switch 31 (first switch 32, second switch 33) of the hand piece 4 and the output set value in each output mode are set and displayed on the operation display panel 46 of the ultrasonic driving unit 2 and the operation display panel 53 of the high frequency driving unit 3.

FIG. 11 shows an example of output modes set on the operation display panel 46 and operation display panel 53. Here, No. 2 shown in FIG. 6 is selected as an output mode by the first operation of the first switch 32 (the first output mode of the first switch 32), and No. 3 shown in FIG. 6 is selected as an output mode by the second operation (the second output mode of the first switch 32). No. 11 shown in FIG. 6 is selected as an output mode by the first operation of the second switch 33 (the first output mode of the second switch 33), and No. 12 shown in FIG. 6 is selected as an output mode by the second operation (the second output mode of the second switch 33).

FIG. 12 shows another example of output modes set on the operation display panel 46 and operation display panel 53. Here, No. 16 shown in FIG. 6 is selected as a first output mode of the first switch 32, and No. 1 shown in FIG. 6 is selected as a second output mode. No. 16 shown in FIG. 6 is selected as a first output mode of the second switch 33, and No. 10 shown in FIG. 6 is selected as a second output mode.

For the other output modes than those shown in FIG. 11 and FIG. 12, the first and second output modes of the first switch 32 and the first and second output modes of the second switch 33 can be optionally selected from the output modes shown in FIG. 6.

The number of operation switches is not limited to the above first switch 32 and second switch 33. More than three operation switches may be provided, and more than three output modes may be set by each operation switch.

FIG. 13A - FIG. 15B show the configuration of the hand switch 31. FIG. 13A, FIG. 14A and FIG. 15A show the external view of the hand pieces 4. FIG. 13B, FIG. 14B and FIG. 15B show the circuit diagram explaining the operation of the first switch 32. The first switch 32 and second switch 33 are configured as a pushbutton type two-step operation switch. FIG. 13A shows the state that the first switch 32 is not pressed (not operated). In this time, as shown in FIG. 13B, the terminals A, B and C are electrically opened. FIG. 14A shows the state that the first switch 32 is pressed to the first operation position (first operation). In this time, as shown in FIG. 14B, the terminals A and B are electrically shorted. FIG. 15A shows the state that the first switch 32 is pressed from the first operation position to the second operation position (second operation). In this time, as shown in FIG. 15B, the terminals A, B and C are electrically shorted. A chain double-dashed line in FIG. 14A and FIG. 15A indicates the state of the first switch 32 in FIG. 13A (the state that the first switch 32 is not pressed). The operator can easily confirm the operation state (no operation, first operation or second operation) by the click noise when the first switch 32 is pressed.

The electric signal to open or short the terminals A, B and C is sent to the CPU 45 of the ultrasonic driving unit 2 through the conductor 35 of the switch connection cable 7. Therefore, when the first operation shown in FIG. 14A is performed (the terminals A and B are shorted), the previously set first output mode of the first switch 32 is selected. When the second operation shown in FIG. 15A is operated (the terminals A, B and C are shorted), the previously set second output mode of the first switch is selected.

In FIG. 13A - FIG. 15B, only the first switch 32 is explained. The configuration of the second switch 33 is the same as the first switch 32. Therefore, when the first operation of the second switch 33 is performed, the previously set first output mode of the second switch 33 is selected. When the second operation is performed, the previously set second output mode of the second switch 33 is selected.

Here, the first and second switches 32 and 33 are pushbutton type two-step operation switches. But, the switch is not limited to this type. A locker type or slide type two-step operation switch may be used.

(Function)

An explanation will be given on treatment of a living tissue by using the treatment apparatus for operation 1. First, apply the patient plate 9 tightly to a living tissue. Set the output condition of the treatment apparatus for operation 1. The output condition is set and displayed on the operation display panel 46 of the ultrasonic driving unit 2 and the operation display panel 53 of the high frequency driving unit 3. As an output condition, there are four output modes: first and second output modes of the first switch 32, and first and second output modes of the second switch 33 (refer to FIG. 11 and FIG. 12). Set the output set value in each output mode on the operation display panel 46 and operation display panel 53. The set output condition is displayed on the operation display panel 46 and operation display panel 53. Apply the treatment unit 26 of the hand piece 4 to a treatment object area of a living tissue. Perform the first operation of the first switch 32 of the hand switch 31 (refer to FIG. 14) to drive the hand piece 4 in the first output mode of the first switch 32. Perform the second operation of the first switch 32 (refer to FIG. 15) to drive the hand piece 4 in the second output mode of the first switch 32. Perform the first operation of the second switch 33 of the hand switch 31 to drive the hand piece 4 in the first output mode of the second switch 33. Perform the second operation of the second switch 33 to drive the hand piece 4 in the second output mode of the second switch 33. The living tissue contacting the treatment unit 26 of the hand piece 4 is treated by ultrasonic output and/or high-frequency output.

Functions in each output condition will be explained. Explanation will be given first on the output condition combining Off of high-frequency output, On of ultrasonic output or Max of ultrasonic output (No. 6 or No. 17 in FIG. 6). Only an ultrasonic output is obtained in this condition. When the hand switch 31 is operated, the ultrasonic transducer 12 of the hand piece 4 is driven by the ultrasonic transducer driving circuit 37. The probe 25 is ultrasonically activated, and the living tissue contacting the treatment unit 26 of the probe 25 is coagulated and cut by frictional heat generated by ultrasonic activation. On and Max of ultrasonic output are kept constant (amplitude of ultrasonic activation). A current control system is adopted as an amplitude control system in this embodiment, and the ultrasonic transducer 12 is driven by constant-current control. Namely, the same treatment as that performed by a conventional ultrasonic coagulation/cutting apparatus is performed.

Explanation will now be given on the output condition combining each output mode of high-frequency output and Off of ultrasonic output (No. 2, No. 5, No. 8, No. 11 or No. 14 in FIG. 6). Only a high-frequency output is obtained in this condition. When the hand switch 31 is operated, a control signal of the hand switch 31 is sent from the CPU 45 of the ultrasonic transducer driving circuit 37 to the output controller 52 of the high frequency driving circuit 48. The high frequency driving circuit 48 is driven, and a high-frequency current flows over the treatment unit 26 of the hand piece 4 and the patient plate 9. The living tissue contacting the treatment unit 26 is coagulated and cut by Joule heat generated by the high-frequency current. Namely, the same treatment as that executed by a conventional monopolar electric surgical knife is performed.

Explanation will be given on the output condition combining each output mode of high-frequency output and On of ultrasonic output (No. 1, No. 4, No. 7, No. 10 or No. 13 in FIG. 6). High-frequency output and ultrasonic output are simultaneously obtained in this condition. When the hand switch 31 is operated, the ultrasonic transducer 12 of the hand piece 4 is driven by the ultrasonic transducer driving circuit 37, and the probe 25 is ultrasonically activated. A control signal of the hand switch 31 is sent to the output controller 52 of the high frequency driving circuit 48, and a high-frequency current flows over the treatment unit 26 of the hand piece 4 and the patient plate 9. A living tissue contacting the treatment unit 26 is treated by both ultrasonic output and high-frequency output. The ultrasonic transducer 12 is driven by constant-current control, and the ultrasonic output is kept at a previously set value (e.g. 70%). The output characteristics of high-frequency output are as shown in FIG. 7 and FIG. 8.

Namely, when the electric impedance of a living tissue is relatively low, the set high-frequency output is kept. But, when the impedance is increased to relatively high, the high-frequency output is decreased. Therefore, when the electric impedance of a living tissue is relatively low (e.g. when a lot of blood is stuck to a living tissue), previously set an ultrasonic output to low (e.g. 30%). Then sufficient coagulation and cutting are possible with the high-frequency output, and the influence of cavitation from the probe distal end by an ultrasonic output (e.g. splash of blood) can be controlled to minimum.

When the electric impedance of a living tissue is high (e.g. when a living tissue is very dried), previously set an ultrasonic output to high (e.g. 100%). Then even if a high-frequency output is decreased, sufficient coagulation and cutting are possible by the action of ultrasonic output. In either case (when the ultrasonic output is low or high), the treatment unit 26 of the probe 25 is ultrasonically activated, and adhesion and burning of a living tissue to the treatment unit 26 are prevented, and coagulation and cutting can be always kept sufficient.

Explanation will be given on the output condition combining each output mode of high-frequency output and Auto of ultrasonic output (No. 3, No. 6, No. 9, No. 12 or No. 15 in FIG. 6). High-frequency output and ultrasonic output are simultaneously obtained in this condition, and the largeness of ultrasonic output is changed interlocking with the largeness of impedance in the high-frequency output. When the hand switch 31 is operated, the ultrasonic transducer 12 of the hand piece 4 is driven by the ultrasonic transducer driving circuit 37, and the probe 25 is ultrasonically activated. A control signal of the hand switch 31 is sent to the output controller 52 of the high frequency driving circuit 48, and a high-frequency current flows over the treatment unit 26 of the hand piece 4 and the patient plate 9. A living tissue contacting the treatment unit 26 is treated by both ultrasonic output and high-frequency output. In this time, a signal indicating the largeness of impedance in the high frequency output is sent from the output controller 52 of the high frequency driving circuit 48 to the CPU 45 of the ultrasonic transducer driving circuit 37. Based on this signal, the CPU 45 instructs the ultrasonic output condition shown in FIG. 10 to the D/A converter 44. The D/A converter 44 generates a signal indicating the largeness of current corresponding to the ultrasonic output condition instructed by the CPU 45. The largeness of ultrasonic output (amplitude of ultrasonic activation) is changed interlocking with the largeness of impedance in the ultrasonic output. The output characteristics of high-frequency output of this time are as shown in FIG. 7 and FIG. 8. Namely, when the electric impedance of a living tissue is relatively low, a previously set high-frequency output is kept. But, when the impedance is relatively high, the high-frequency output is decreased. As an example, FIG. 16 shows the output characteristics in the output condition (combining Coag-Hard of high-frequency output and Auto of ultrasonic output) indicated by No. 12 in FIG. 6.

FIG. 16 shows an example of setting the set value of high-frequency output Coag-Hard to 50W. As shown in FIG. 16, when the electric impedance of a living tissue is lower than approximately 2 kΩ (e.g. when a lot of blood is stuck to a living tissue), the high-frequency output becomes 50W and the ultrasonic output becomes 30%. Therefore, sufficient coagulation and cutting are possible with the high-frequency output, and the influence of cavitation from the probe distal end by an ultrasonic output (e.g. splash of blood) can be controlled to minimum. When the electric impedance of a living tissue is higher than approximately 4 kΩ (e.g. when a living tissue is very dried), the high-frequency output becomes lower than approximately 30W and the ultrasonic output becomes 100%. Therefore, even if a high-frequency output is decreased, sufficient coagulation and cutting are possible by the action of ultrasonic output. When the electric impedance of a living tissue is medium in a range of 2 kΩ - 4 kΩ (when a living tissue is in a normal state), the high-frequency output becomes 50W - 30W and the ultrasonic output becomes 70%. Therefore, sufficient coagulation and cutting are possible by the action of high-frequency output and ultrasonic output. In either case, the treatment unit 26 of the probe 25 is ultrasonically activated, and adhesion and burning of a living tissue to the treatment unit 26 are prevented, and coagulation and cutting can be always kept satisfactory. The largeness of high-frequency output and ultrasonic output is automatically changed according to the electric impedance of a living tissue to be treated (i.e. the kind and state of a living tissue to be treated). Therefore, it is unnecessary to set the high-frequency output and ultrasonic output according to the kind and state of a living tissue to be treated, and the operation becomes easy.

(Effects)

According to this embodiment, four output modes can be optionally selected from the output modes shown in FIG. 6, and set as an output condition. Therefore, various operations and treatments favorite to the operator are possible, and versatility is increased.

Outputs in four different output modes can be selectively obtained by operating two switches (first switch 32 and second switch 33). Further, as the first switch 32 and second switch 33 are two-step operation switches and intuitive to use, the operator can easily perform treatment in a desired output mode. Namely, the operator can easily perform treatment in more than two output modes with fewer switches than the number of output modes, and operability is improved.

(Embodiment 2)

A second embodiment of the invention will be explained. Only the parts different from the first embodiment will be explained.

(Configuration)

A hand piece 55 of this embodiment is a scissors-like surgical instrument. The hand piece 55 has both functions as a bipolar electric surgical knife and an ultrasonic coagulation/cutting instrument.

FIG. 17 shows the configuration of the hand piece 55 of the second embodiment. A treatment unit 57 is provided at the distal end of a slender sheath 56, and a control unit 58 is provided at the proximal end. The control unit 58 is provided with a case 59 for housing a not-shown ultrasonic transducer for generating ultrasonic activation, and an operation handle 60. Inside the sheath 56, a probe 61 is provided for transmitting the ultrasonic activation from the ultrasonic transducer to the treatment unit 57. The probe 61 is made of metallic material such as titanium, duralumin, and stainless steel. The distal end of the probe 61 is exposed outside the distal end of the sheath 56. The treatment unit 57 is provided with a grasping unit 63 which is opened and closed to the distal end exposed portion 62. The grasping unit 63 is connected to the distal end of the sheath 56 rotatably about a pivot pin 64. The grasping unit 63 is opened and closed to the distal end exposed portion 62 by the operation of the operation handle 60, and a living tissue can be grasped between the probe 61 and grasping unit 63. A part of the grasping unit 63 is made of metallic material, and electrically insulated from the probe 61.

The rear end of the case 59 is connected with one end of the output connection cable 6 shown in the first embodiment. The other end of the output connection cable 6 is connected to the ultrasonic driving unit 2 shown in the first embodiment. Therefore, the power for ultrasonic driving is supplied to the ultrasonic transducer.

The control unit 58 is provided with a first switch 66 and a second switch 67 as a hand switch 65. The first switch 66 and second switch 67 are configured as a pushbutton type two-step operation switch as in the first embodiment. The rear end of the case 59 is connected with one end of the switch connection cable 7 shown in the first embodiment, and connected to the hand switch 65 as in the first embodiment. The other end of the switch connection cable 7 is connected to the ultrasonic driving unit 2 shown in the first embodiment. Therefore, when the first operation of the first switch 66 is performed, the first output mode of the first switch 66 is selected. When the second operation is performed, the previously set second output mode of the first switch 66 is selected. When the first operation of the second switch 67 is performed, the previously set first output mode of the second switch 67 is selected. When the second operation is performed, the previously set second output mode of the second switch 67 is selected.

The rear end of the case 59 is connected to one end of the output connection cable 68. Two insulated conductors (not shown) are provided inside the output connection cable 68. One of the two conductors is electrically continued to the probe 61, and the other conductor is electrically continued to the metallic material part of the grasping unit 63. The other end of the output connection cable 68 is connected to the high frequency driving unit 3 shown in the first embodiment. Therefore, the hand piece 55 functions as a bipolar electric surgical knife which treats a living tissue grasped between the probe 61 and grasping unit 63 with a high-frequency current. The output connection cable 68 is composed of the output connection cable 8 and connection cable 10 shown in the first embodiment as one piece, and the patient plate 9 shown in the first embodiment becomes unnecessary.

FIG. 18 is a table showing a list of output modes in this embodiment. Like a conventional bipolar electric surgical knife, the high frequency driving unit 3 enables high-frequency output in each mode of Cut-Pure (cut output including almost no hemostatic component), Coag-Soft (coagulation output for stopping the bleeding of tissue), and Coag-Hard (coagulation output for sealing pulse vessels such as blood vessels). The ultrasonic driving unit 2 enables ultrasonic output in each mode of On (ultrasonic output keeping a set value of amplitude of ultrasonic activation), and Auto (ultrasonic output changing the amplitude of ultrasonic activation interlocking with the largeness of impedance in the high-frequency output).

In this embodiment, the output modes of the high-frequency output and ultrasonic output can be combined. It is also possible to combine Off of high-frequency output (no output), On of ultrasonic output (ultrasonic output keeping a set value of amplitude of ultrasonic activation) or Max of ultrasonic output (ultrasonic output keeping a maximum value of amplitude of ultrasonic activation) (only ultrasonic output is obtained). It is also possible to combine the output modes of high-frequency output (e.g. Cut-Pure and Coag-Hard) and Off of ultrasonic output (no output) (only high-frequency output is obtained).

The output modes corresponding to the hand switch 65 (first switch 66 and second switch 67) of the hand piece 55, and the output set values in each output mode are set and displayed on the operation display panel 46 of the ultrasonic driving unit 2 and the operation display panel 53 of the high frequency driving unit 3, as in the first embodiment.

FIG. 19 shows an example of output modes set on the operation display panels 46 and 53. Here, No. 10 shown in FIG. 18 is selected as an output mode by the first operation of the first switch 66 (the first output mode of the first switch 66), and No. 11 shown in FIG. 18 is selected as an output mode by the second operation (the second output mode of the first switch 66). No. 8 shown in FIG. 18 is selected as an output mode by the first operation of the second switch 67 (the first output mode of the second switch 67), and No. 7 shown in FIG. 18 is selected as an output mode by the second operation (the second output mode of the second switch 67).

Other than those shown in FIG. 19, the first and second output modes of the first switch 66 and the first and second output modes of the second switch 67 can be optionally selected from the output modes shown in FIG. 18.

(Function)

An explanation will be given on treatment of a living tissue by using the treatment apparatus for operation of this embodiment. First, set the output condition of the treatment apparatus for operation. As in the first embodiment, there are four output modes: first and second output modes of the first switch 66, and first and second output modes of the second switch 67 (refer to FIG. 19). Set the output set value of each output mode. Then, place a living tissue between the grasping unit 63 and the probe 61 of the hand piece 55. Operate the handle 60 of the control unit 58 in the closing direction, and grasp the living tissue between the grasping unit 63 and probe 61. Perform the first operation of the first switch 66 of the hand switch 65 to drive the hand piece 55 in the first output mode of the first switch 66. Perform the second operation of the first switch 66 to drive the hand piece 55 in the second output mode of the first switch 66. Perform the first operation of the second switch 67 of the hand switch 65 to drive the hand piece 55 in the first output mode of the second switch 67. Perform the second operation of the second switch 67 to drive the hand piece 55 in the second output mode of the second switch 67. The living tissue grasped between the grasping unit 63 and the probe 61 of the hand piece 55 is treated by ultrasonic output and/or high-frequency output.

Functions in each output condition will be explained. Explanation will be given first on the output condition combining Off of high-frequency output, On of ultrasonic output or Max of ultrasonic output (No. 10 or No. 11 in FIG. 18). Only an ultrasonic output is obtained in this condition. Therefore, as in a conventional ultrasonic coagulation/cutting apparatus, the living tissue grasped between the grasping unit 63 and probe 61 is coagulated and cut by frictional heat generated by ultrasonic activation.

Explanation will now be given on the output condition combining each output mode of high-frequency output and Off of ultrasonic output (No. 2, No. 5 or No. 8 in FIG. 18). Only a high-frequency output is obtained in this condition. When the hand switch 65 is operated, a high-frequency current flows over the grasping unit 63 and the probe 61 of the hand piece 55. The living tissue grasped between the grasping unit 63 and probe 61 is coagulated and cut by Joule heat generated by the high-frequency current. Namely, the same treatment as that performed by a conventional bipolar electric surgical knife is performed.

Explanation will be given on the output condition combining each output mode of high-frequency output and On or Auto of ultrasonic output (No. 1, No. 3, No. 6, No. 7 or No. 9 in FIG. 18). High-frequency output and ultrasonic output are simultaneously obtained in this condition. When the hand switch 65 is operated, the ultrasonic transducer of the hand piece 55 is driven by the ultrasonic transducer driving circuit 37, and the probe 61 is ultrasonically activated. A control signal of the hand switch 65 is sent to the output controller 52 of the high frequency driving circuit 48, and a high-frequency current flows over the grasping unit 63 and probe 61. The living tissue grasped between the grasping unit 63 and probe 61 is treated by both ultrasonic output and high-frequency output.

(Effects)

According to this embodiment, a living tissue is treated by both frictional heat generated by ultrasonic activation and Joule heat generated by high-frequency current, and coagulated and cut more effectively than treatment only by ultrasonic output or high-frequency output.

(Embodiment 3)

A third embodiment of the invention will be explained. Only the parts different from the first embodiment will be explained.

(Configuration)

FIG. 20 shows the configuration of a treatment apparatus for operation 71 according to a third embodiment. The treatment apparatus for operation 71 comprises a hand piece 73 and a foot switch 74. The hand piece 73 is connected to an ultrasonic/high frequency driving unit 72 through an output/switch connection cable 75. The foot switch 74 is connected to the ultrasonic/high frequency driving unit 72 by a switch connection cable 76. The ultrasonic/high frequency driving unit 72 is connected to the patient plate 9 by the connection cable 10.

The hand piece 73 of this embodiment functions as an ultrasonic suction treatment unit. The hand piece 73 is provided with a probe 77 and a sheath 78 covering the probe 77. The probe 77 is made of made of metallic material such as titanium, duralumin, and stainless steel. The probe 77 is cylindrical, and its internal cavity is formed as a part of a suction channel. A suction channel is formed on the center axis of the hand piece 73 up to the rear end of the hand piece 73. The suction channel is communicatively connected to a suction tube 79 connected to the rear end of the hand piece 73. A clearance formed between the probe 77 and sheath 78 forms a water supply channel. This water supply channel is communicatively connected to a water supply tube 80 connected to the rear end of the hand piece 73. The water supply tube 80 is connected to a not-shown water supply unit. Therefore, suction from the suction channel of the hand piece 73 and water supply to the water supply channel are possible. As in the first embodiment, the treatment unit 81 of the hand piece 73 is configured to receive ultrasonic activation and high-frequency current. The hand piece 73 is provided with a first switch 83 and a second switch 84 as a hand switch 82. The first and second switches 83 and 84 are configured as a pushbutton type two-step operation switch.

Inside the ultrasonic/high frequency driving unit 72, the ultrasonic transducer driving circuit 37 and high frequency driving circuit 48 shown in FIG. 5 of the first embodiment are provided. As in the first embodiment, the CPU 45 of the ultrasonic transducer driving circuit 37 and the output controller 52 of the high frequency driving circuit 48 are connected to transmit a signal in two ways. The ultrasonic/high frequency driving unit 72 is provided with an operation display panel 85. Namely, the ultrasonic/high frequency driving unit 72 includes the ultrasonic driving unit 2 and high frequency driving unit 3 shown in the first embodiment as one unit.

The output/switch connection cable 75 includes the output connection cable 6, switch connection cable 7 and output connection cable 8 shown in the first embodiment as one piece.

The foot switch 74 is provided with a first pedal switch 86 and a second pedal switch 87. The first and second pedal switches 86 and 87 are configured as a foot-operated two-step switch. The switch connection cable 76 is connected to the CPU 45 of the ultrasonic transducer driving circuit 37.

The not-shown suction unit and water supply unit are also connected to the CPU 45 of the ultrasonic transducer driving circuit 37. Therefore, a control signal of the hand switch 82 or foot switch 74 and an output condition set on the operation display panel 85 are sent to the suction unit and water supply unit.

FIG. 21 shows a list of output modes of the treatment apparatus for operation 71. No. 1 - No. 15 in FIG. 21 are the same output modes as No. 1 - No. 15 in FIG. 6 shown in the first embodiment. Namely, in the output modes No. 1 - No. 15 in FIG. 21, a living tissue contacting the treatment unit 81 of the hand piece 73 are coagulated and cut by high-frequency output, or by both high-frequency output and ultrasonic output.

No. 16 and No. 17 in FIG. 21 are output modes for ultrasonic suction. In the output modes No. 16 and No. 17, the probe 77 is ultrasonically activated, and a living tissue contacting the treatment 81 of the probe 77 is emulsified and shattered. At the same time, physiological saline is supplied to the water supply channel of the hand piece 73. The emulsified and shattered living tissue is sucked through the suction channel of the hand piece 73. In the output mode No. 16, an ultrasonic output is On, and a set value of amplitude of ultrasonic activation is kept. In the output mode No. 17, an ultrasonic output is Max, and a maximum value of amplitude of ultrasonic activation is kept. The water supply speed to the water supply channel and suction speed from the suction channel are Auto, and changed to an optimum value interlocking with a set value of ultrasonic output.

No. 18 and No. 19 in FIG. 21 are output modes for suction. In the output modes No. 18 and No. 19, blood in a treating area is sucked from the treatment unit 81 of the probe 77 to the suction unit through the suction channel of the hand piece 73. In the output mode No. 18, suction is Low, and a suction speed is relatively slow. In the output mode No. 19, suction is High, and a suction speed is relatively fast. No. 20 and No. 21 in FIG. 21 are output modes for supplying water. In the output modes No. 20 and No. 21, physiological saline is supplied from the water supply unit to the treatment unit 81 of the probe 77 through the water supply channel, and blood in the treating area is removed. In the output mode No. 20, water supply is Low, and a water speed is relative slow. In the output mode No. 21, water supply is High, and a water supply speed is relatively fast. Namely, in the output modes No. 18 - No. 21 in FIG. 21, the treatment apparatus functions as a water supply/suction unit without ultrasonic output and high-frequency output.

Output modes corresponding to the hand switch 82 (first switch 83 and second switch 84) of the hand piece 73 and the foot switch 74 (first pedal switch 86 and second pedal switch 87), and output set values in each output mode are set and displayed on the operation display panel of the ultrasonic/high frequency driving unit 72.

FIG. 22 shows an example of output modes set on the operation display panel 85. Namely, the following output modes are selected for the hand switch 82. No. 20 shown in FIG. 21 is selected as a first output mode of the first switch 83, and No. 21 shown in FIG. 21 is selected as a second output mode of the first switch 83. No. 18 shown in FIG. 21 is selected as a first output mode of the second switch 84, and No. 19 shown in FIG. 21 is selected as a second output mode of the second switch 84. The following output modes are selected for the foot switch 74. No. 16 shown in FIG. 21 is selected as a first output mode of the first pedal switch 86, and No. 17 shown in FIG. 21 is selected as a second output mode of the first pedal switch 86. No. 11 shown in FIG. 21 is selected as a first output mode of the second pedal switch 87, and No. 12 shown in FIG. 21 is selected as a second output mode of the second pedal switch 87. Namely, in the setting shown in FIG. 22, the hand switch 82 controls the outputs of water supply and suction, and the foot switch 74 controls the outputs of ultrasonic suction and coagulation/cutting of tissue.

Other than those shown in FIG. 22, the output modes of the hand switch 82 and foot switch 74 can be optionally selected from the output modes shown in FIG. 21.

(Function)

An explanation will be given on treatment of a living tissue by using the treatment apparatus for operation 71. First, apply the patient plate 9 tightly to a living tissue. Set the output condition of the treatment apparatus for operation 71. The output condition is set and displayed on the operation display panel 85 of the ultrasonic/high frequency driving unit 72. As an output condition, there are eight output modes: first and second output modes of the first switch 83, first and second output modes of the second switch 84, first and second output modes of the first pedal switch 86, and first and second output modes of the second pedal switch 87 (refer to FIG. 22). Set the output set value in each output mode on the operation display panel 85. Apply or approach the treatment unit 81 of the hand piece 73 to a treatment object area of a living tissue. Perform the operation of the first switch 83 of the hand switch 82 to drive the hand piece 73 in the first output mode of the first switch 83. Perform the second operation of the first switch 83 to drive the hand piece 73 in the second output mode of the first switch 83. Perform the first operation of the second switch 84 of the hand switch 82 to drive the hand piece 73 in the first output mode of the second switch 84. Perform the second operation of the second switch 84 to drive the hand piece 73 in the second output mode of the second switch 84. Perform the first operation of the first pedal switch 86 of the foot switch 74 to drive the hand piece 73 in the first output mode of the first pedal switch 86. Perform the second operation of the first pedal switch 86 to drive the hand piece 73 in the second output mode of the first pedal switch 86. Perform the first operation of the second pedal switch 87 of the foot switch 74 to drive the hand piece 73 in the first output mode of the second pedal switch. Perform the second operation of the second pedal switch 87 to drive the hand piece 73 in the second output mode of the second pedal switch 87. The living tissue contacting or approaching close to the treatment unit 81 of the hand piece 73 is treated.

Functions in each output condition will be explained hereinafter. In the output conditions No. 1 - No. 15 shown in FIG. 21, as in the first embodiment, a living tissue is coagulated and cut by high-frequency output or both high-frequency output and ultrasonic output.

In the output conditions No. 16 and No. 17 shown in FIG. 21, when the hand switch 82 or foot switch 74 is operated, the ultrasonic transducer of the hand piece 73 is driven by the ultrasonic transducer driving circuit 37, and the probe 77 is ultrasonically activated. A control signal of the hand switch 82 or foot switch 74 is sent from the CPU 45 of the ultrasonic transducer driving circuit 37 to the water supply unit and suction unit, and water is supplied to the water supply channel of the hand piece 73 and sucked through the suction channel. The ultrasonic output condition set on the operation display panel 85 is also sent from the CPU 45 to the water supply unit and suction unit. The water supply speed and suction speed are automatically controlled interlocking with a set value of ultrasonic output. For example, as a set value of ultrasonic output is increased, the water supply speed and suction speed are increased. By the above functions, a living tissue contacting the treatment unit 81 of the hand piece 73 is emulsified and shattered by ultrasonic activation, and sucked. Namely, the same treatment as that performed by a conventional ultrasonic suction unit is performed.

In the output conditions No. 18 and No. 19 shown in FIG. 21, when the hand switch 82 or foot switch 74 is operated, a control signal of the hand switch 82 or foot switch 74 is sent from the CPU 45 of the ultrasonic transducer driving circuit 37 to the suction unit, and suction is performed through the suction channel of the hand piece 73. In the output conditions No. 20 and No. 21 shown in FIG. 21, when the hand switch 82 or foot switch 74 is operated, a control signal of the hand switch 82 or foot switch 74 is sent from the CPU 45 of the ultrasonic transducer driving circuit 37 to the water supply unit, and water is supplied to the water supply channel of the hand piece 73. Therefore, in the output conditions No. 18 - No. 21 shown in FIG. 21, blood is sucked from a treatment area, and removed by supplying physiological saline to a treatment area. Namely, the same treatment as a conventional water supply/suction unit (without high-frequency output and ultrasonic output) is performed.

(Effects)

According to this embodiment, eight output modes can be optionally selected from the output modes shown in FIG. 21, and set as an output condition. Therefore, various operations and treatments favorite to the operator are possible, and versatility is increased.

Eight different output modes can be selected by operating four switches (first switch 83, second switch 84, first pedal switch 86 and second pedal switch 87) fewer than the number of output modes (here, eight). Further, as the first switch 83, second switch 84, first pedal switch 86 and second pedal switch 87 are two-step operation switches and intuitive to use, the operator can easily perform treatment in a desired output mode. Further, as the first switch 83 and second switch 84 are operated by hand, and the first pedal switch 86 and second pedal switch 87 are operated by foot of the operator, although the number of switches is as many as four, a misoperation by the operator can be decreased.

As described hereinbefore, according to the invention, an output condition of the treatment apparatus for operation 71 can be optionally selected from various output modes, and set as an output condition. Therefore, various operations and treatments favorite to the operator are possible, and versatility is increased.

Further, treatment can be easily performed in more than two output modes by operating switches fewer than the number of output modes, and operability is improved.

## Claims

1. A treatment apparatus (1) for operation operable in a plurality of output modes **characterized by** comprising:

   a hand piece (4) provided with a probe (25) capable of supplying a high-frequency current, and an ultrasonic transducer (12) connected to the probe (25) for ultrasonically activating the probe (25);
a high frequency driving circuit (3) for supplying a high-frequency current to the probe (25);
an ultrasonic transducer driving circuit (2) for driving the ultrasonic transducer (12);
an operation switch (31) for selecting a first output mode and a second output mode; and
a controller (45, 52) which controls a high-frequency output from the high frequency driving circuit (3) and an ultrasonic output from the ultrasonic transducer driving circuit (2), for operating the treatment apparatus (1) for operation in an output mode selected by the operation switch (31).

2. The treatment apparatus for operation according to claim 1, **characterized in that** the operation switch (31) is composed of a two-step operation switch configured to be set to a first operation position and a second operation position.

3. The treatment apparatus for operation according to claim 2, **characterized in that** the controller (45, 52) operates the treatment apparatus (1) for operation in the first output mode when the operation switch (31) is set to the first operation position, and operates the treatment apparatus for operation in the second output mode when the operation switch (31) is set to the second operation position.

4. The treatment apparatus for operation according to claim 3, **characterized in that** the first output mode is a high-frequency output mode to output a high-frequency signal from the high frequency driving circuit (3), or an ultrasonic output mode to output an ultrasonic signal from the ultrasonic transducer driving circuit (2); and the second output mode is an output mode combining the high-frequency output mode and the ultrasonic output mode.

5. The treatment apparatus for operation according to claim 1, **characterized by** further comprising a fluid supply unit (80) for supplying fluid to the hand piece (73), and a suction unit (79) for collecting fluid from the hand piece (73).

6. The treatment apparatus for operation according to claim 5, **characterized in that** the first and second output modes include a fluid supply/suction output mode to control a fluid supply output of the fluid supply unit (80) and/or a suction output of the suction unit (79); and the operation switch (82) includes an operation switch for setting the fluid supply/suction output mode.

7. A hand piece (4) used in a treatment apparatus (1) for operation operable in a plurality of output modes, **characterized by** comprising:

a probe (25) capable of supplying a high-frequency current;
an ultrasonic transducer (12) which is connected to the probe (25), and ultrasonically activates the probe (25); and
an operation switch (31) for selecting a first output mode and a second output mode,

wherein a high-frequency output from a high frequency driving circuit (3) for supplying a high-frequency current to the probe (25), and an ultrasonic output from an ultrasonic transducer driving circuit (2) for driving the ultrasonic transducer (12) are controlled so as to operate the treatment apparatus (1) for operation in an output mode selected by the operation switch (31).

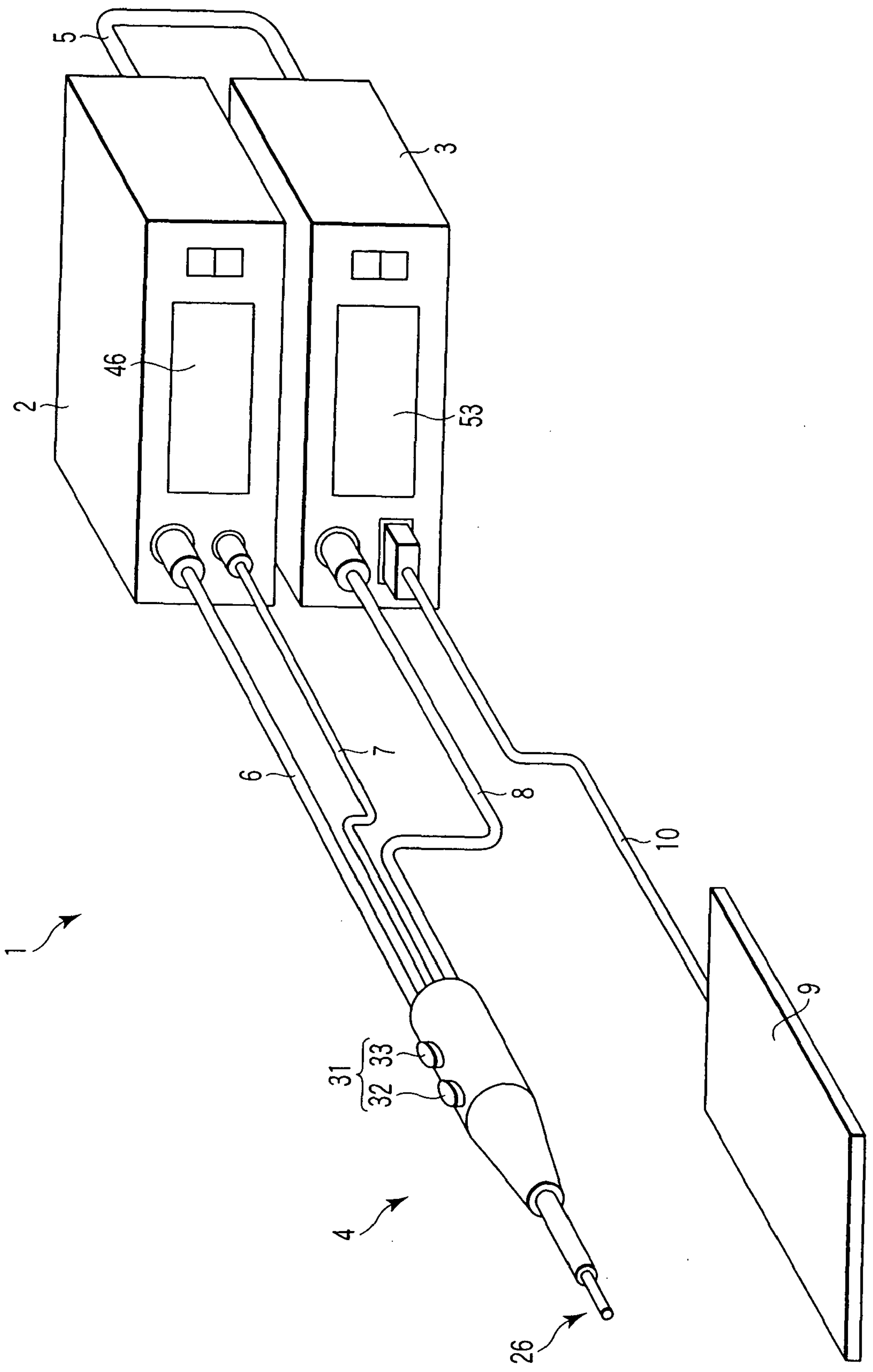


F I G. 1

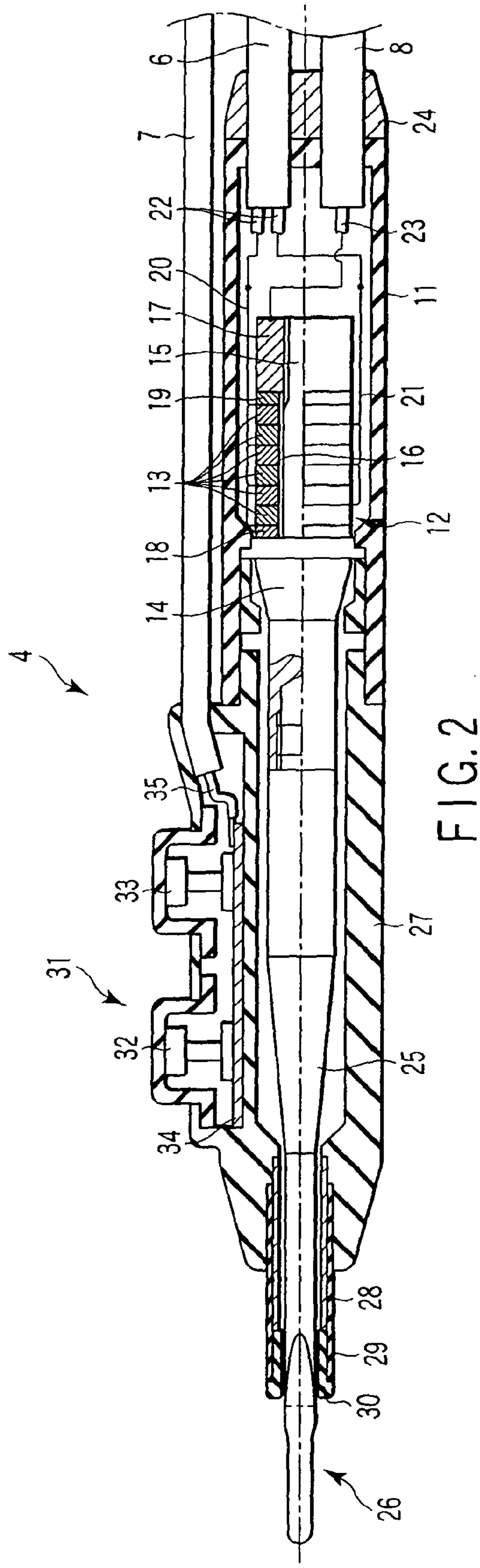


FIG. 2

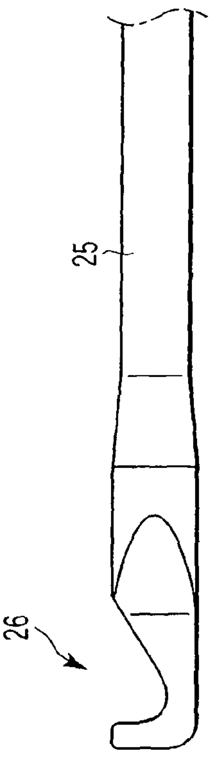


FIG.3

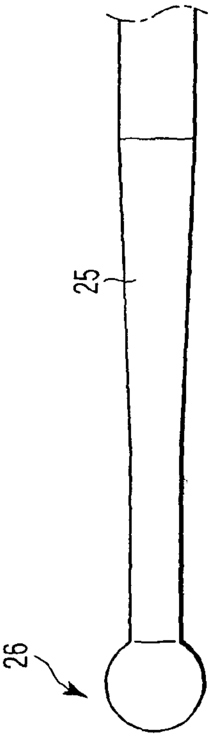


FIG. 4

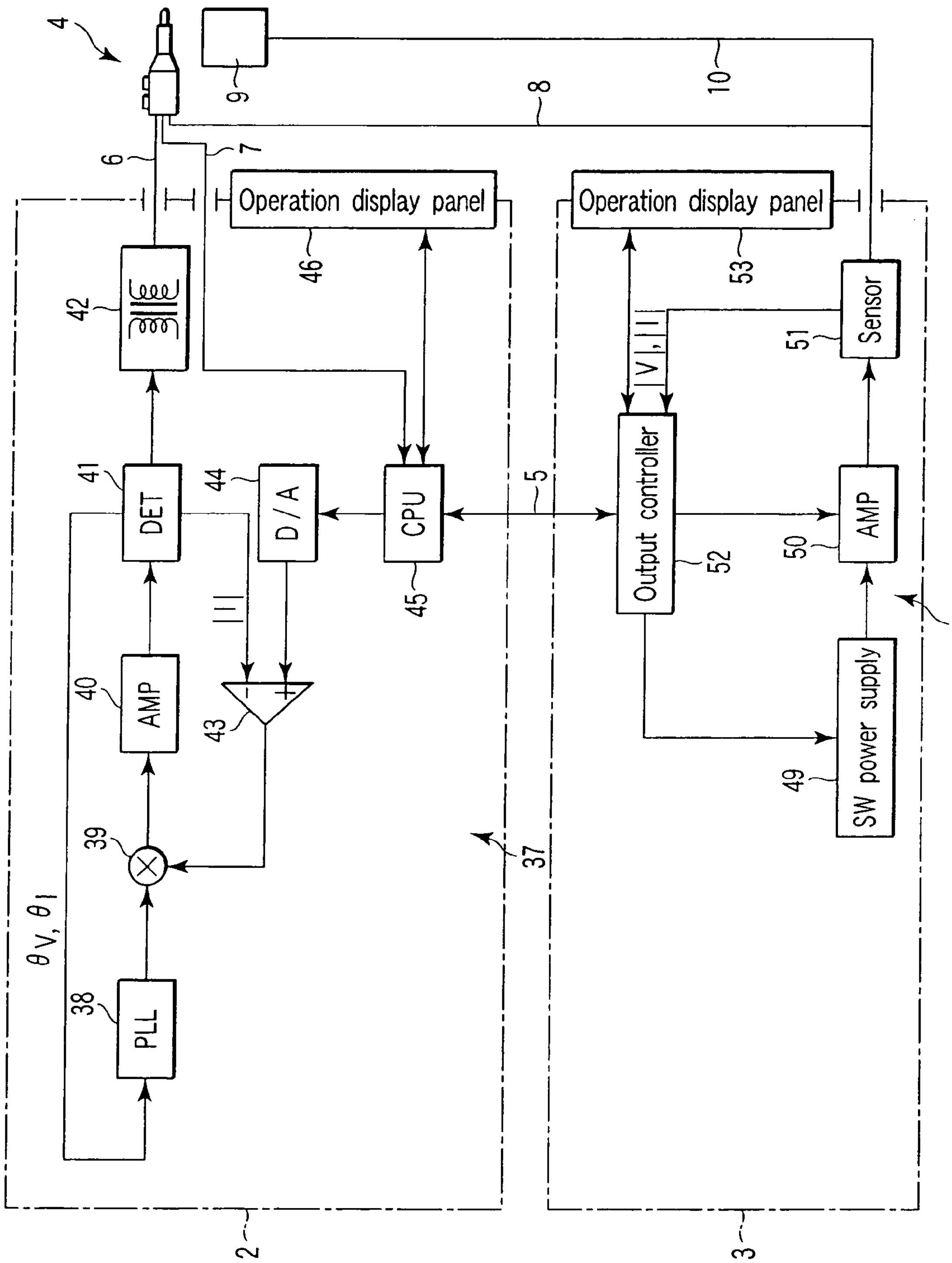

Operation display panel
Operation display panel
DET
D / A
CPU
AMP
PLL
Output controller
Sensor
AMP
SW power supply
|V|, |I|
|I|
θV, θI


FIG. 5

Table of output modes

| High-frequency output | | | Ultrasonic output | No |
|---|---|---|---|---|
| Monopolar | Cut | Pure | On | 1 |
| | | | Off | 2 |
| | | | Auto | 3 |
| | | Blend | On | 4 |
| | | | Off | 5 |
| | | | Auto | 6 |
| | Coag | Soft | On | 7 |
| | | | Off | 8 |
| | | | Auto | 9 |
| | | Hard | On | 10 |
| | | | Off | 11 |
| | | | Auto | 12 |
| | | Spray | On | 13 |
| | | | Off | 14 |
| | | | Auto | 15 |
| Off | | | On | 16 |
| | | | Max | 17 |

FIG. 6

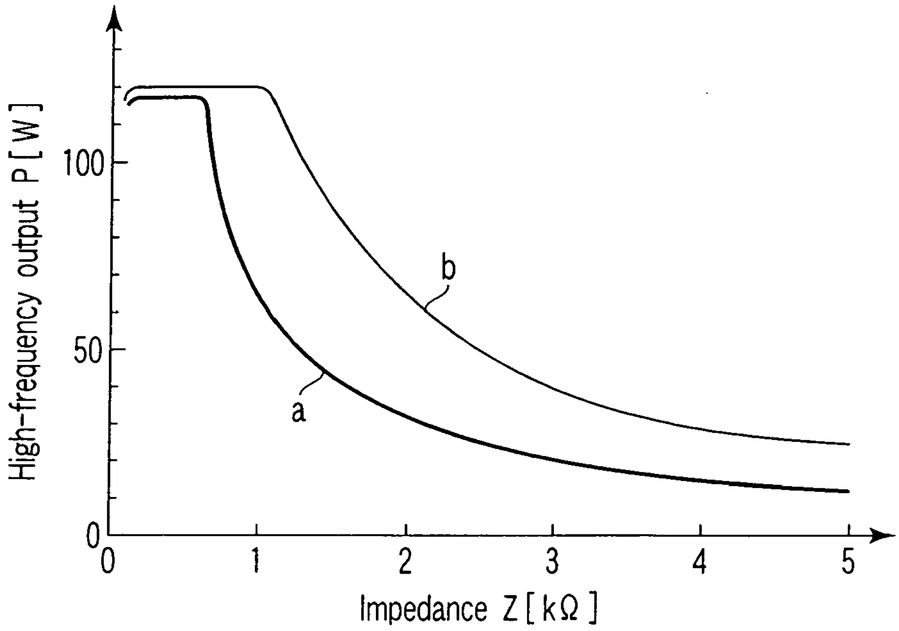
High-frequency output P [ W ]
100
50
0
0
1
2
3
4
5
Impedance Z [ kΩ ]
a
b

FIG. 7

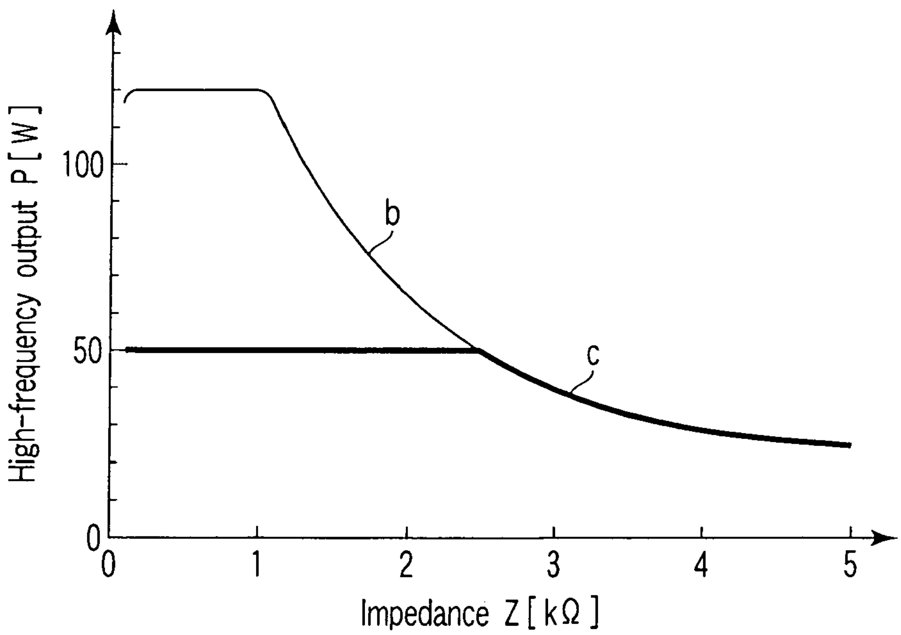
High-frequency output P [ W ]
100
50
0
0
1
2
3
4
5
Impedance Z [ kΩ ]
b
c

FIG. 8

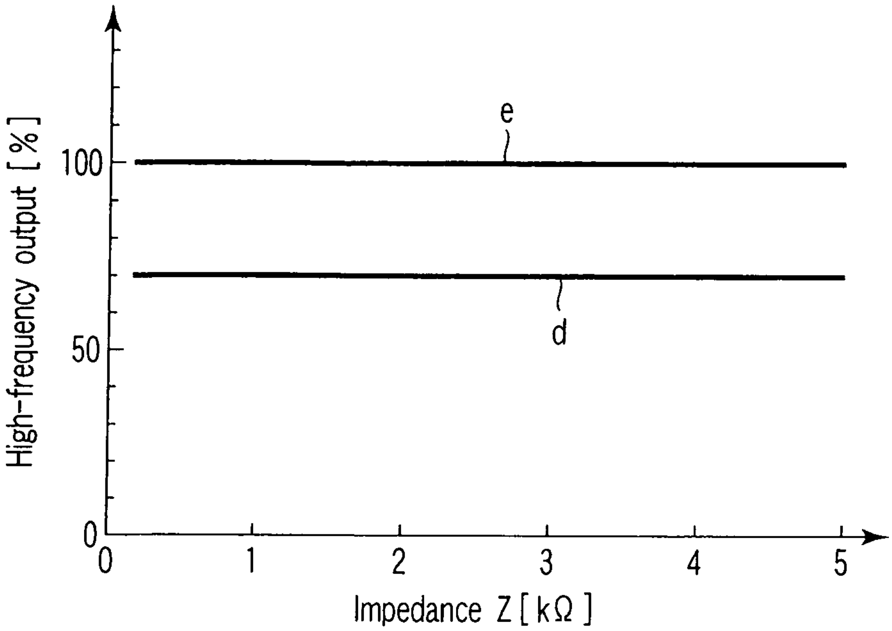
High-frequency output [%]
100
50
0
e
d
0
1
2
3
4
5
Impedance Z [kΩ]

F I G. 9

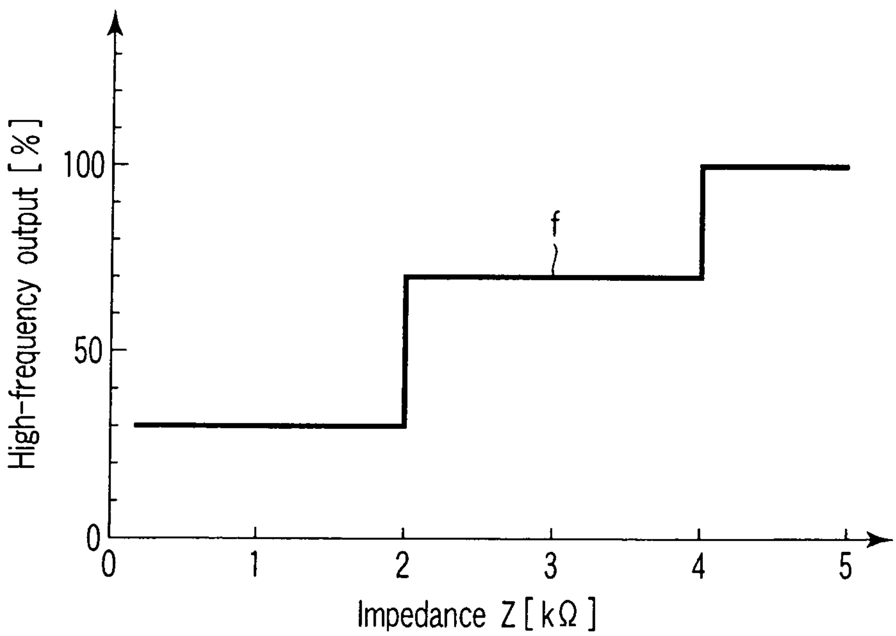
High-frequency output [%]
100
50
0
f
0
1
2
3
4
5
Impedance Z [kΩ]

F I G. 10

| Hand switch | | Output mode |
|---|---|---|
| First switch | First output mode | No2 High-frequency output : Monopolar Cut - Pure<br>Ultrasonic output : Off |
| | Second output mode | No3 High-frequency output : Monopolar Cut - Pure<br>Ultrasonic output : Auto |
| Second switch | First output mode | No11 High-frequency output : Monopolar Coag - Hard<br>Ultrasonic output : Off |
| | Second output mode | No12 High-frequency output : Monopolar Coag - Hard<br>Ultrasonic output : Auto |

FIG. 11

| Hand switch | | Output mode |
|---|---|---|
| First switch | First output mode | No16 High-frequency output : Off<br>Ultrasonic output : On |
| | Second output mode | No1 High-frequency output : Monopolar Cut - Pure<br>Ultrasonic output : On |
| Second switch | First output mode | No16 High-frequency output : Off<br>Ultrasonic output : On |
| | Second output mode | No10 High-frequency output : Monopolar Coag - Hard<br>Ultrasonic output : On |

FIG. 12

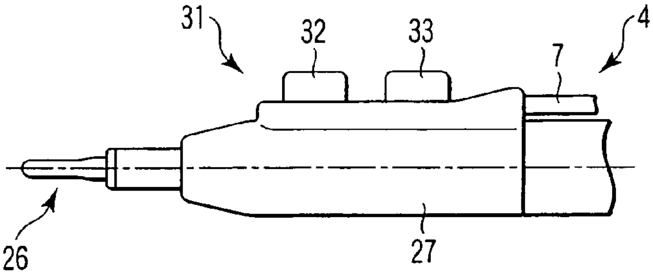


FIG. 13A

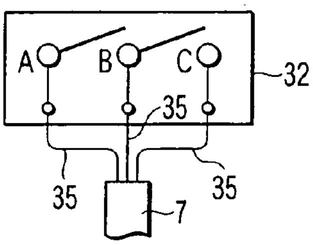


FIG. 13B

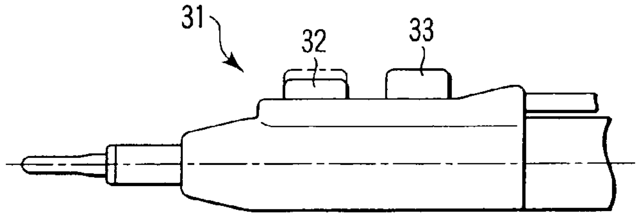


FIG. 14A

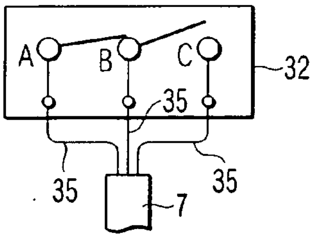


FIG. 14B

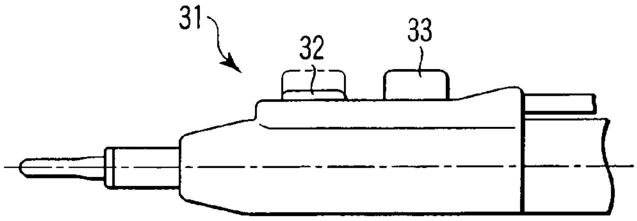


FIG. 15A

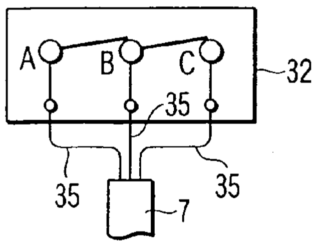


FIG. 15B

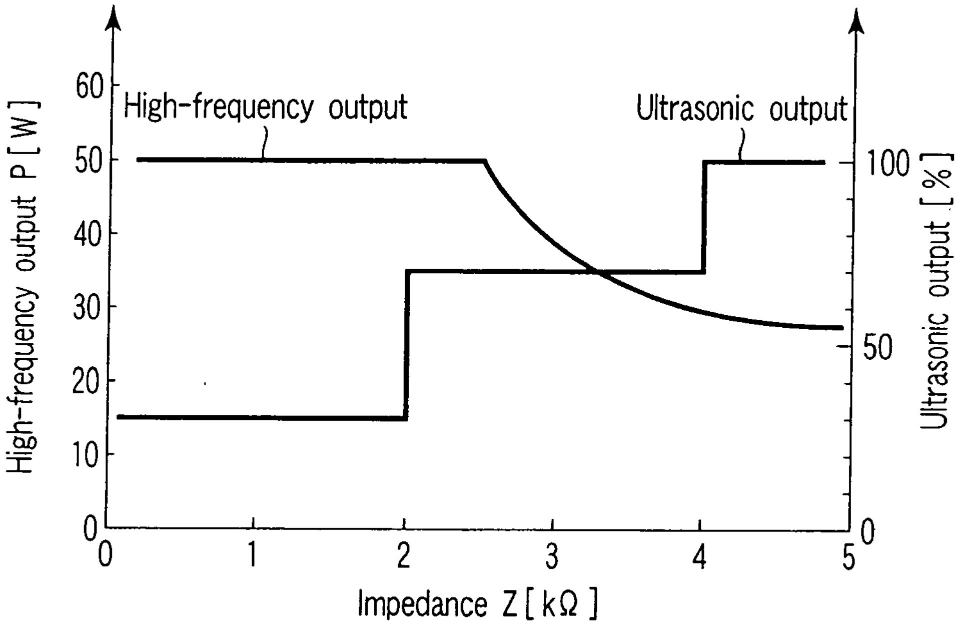
60
50
40
30
20
10
0
High-frequency output P [ W ]
High-frequency output
Ultrasonic output
100
50
0
Ultrasonic output [ % ]
0
1
2
3
4
5
Impedance Z [ kΩ ]

F I G. 16

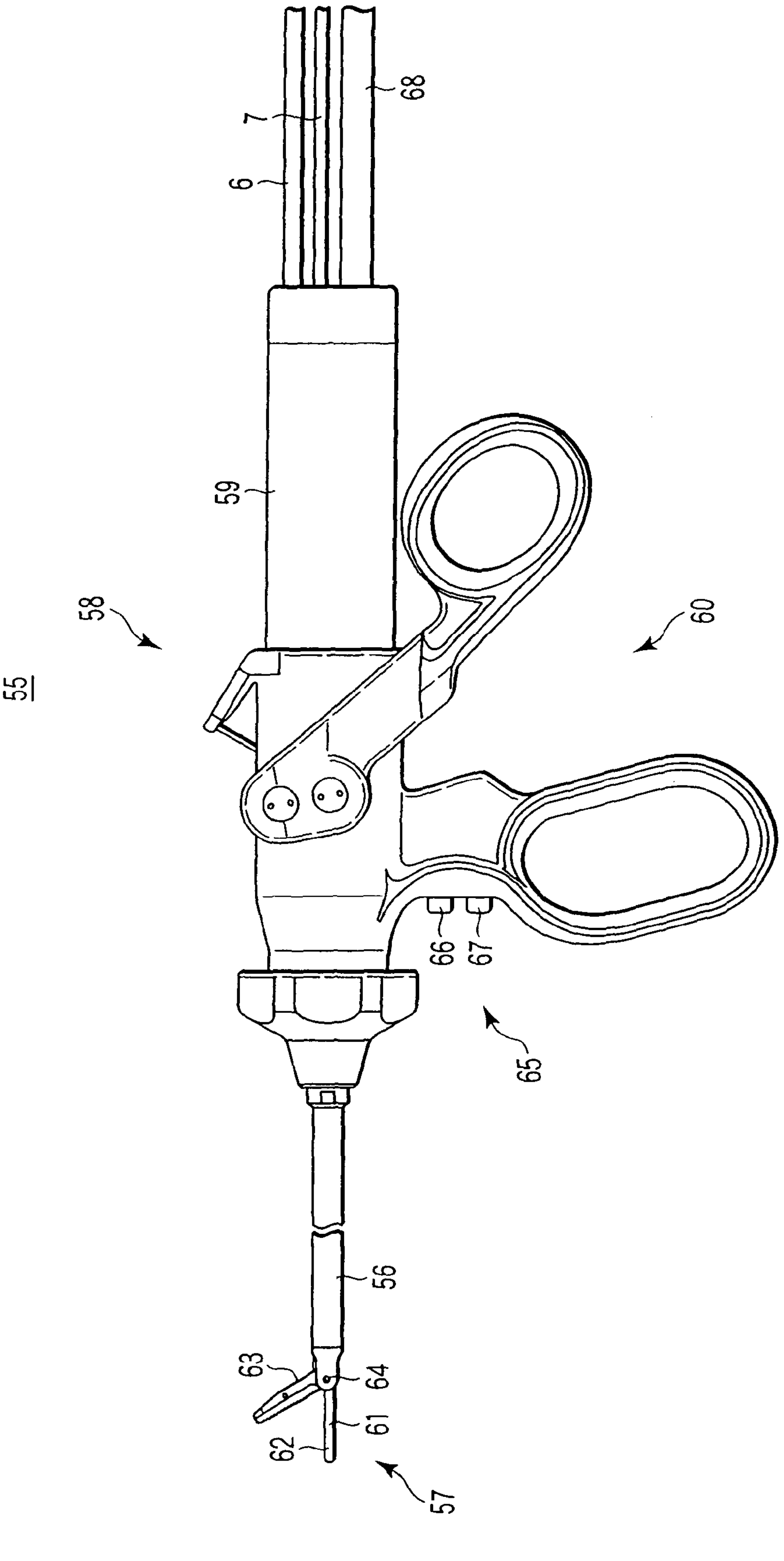


FIG. 17

Table of output modes

| High-frequency output | | | Ultrasonic output | No |
|---|---|---|---|---|
| Bipolar | Cut | Pure | On | 1 |
| | | | Off | 2 |
| | | | Auto | 3 |
| | Coag | Soft | On | 4 |
| | | | Off | 5 |
| | | | Auto | 6 |
| | | Hard | On | 7 |
| | | | Off | 8 |
| | | | Auto | 9 |
| Off | | | On | 10 |
| | | | Max | 11 |

FIG. 18

| Hand switch | | Output mode |
|---|---|---|
| First switch | First output mode | No10 High-frequency output : Off<br>Ultrasonic output : On |
| | Second output mode | No11 High-frequency output : Off<br>Ultrasonic output : Max |
| Second switch | First output mode | No8 High-frequency output : Bipolar Coag - Hard<br>Ultrasonic output : Off |
| | Second output mode | No7 High-frequency output : Bipolar Coag - Hard<br>Ultrasonic output : On |

FIG. 19

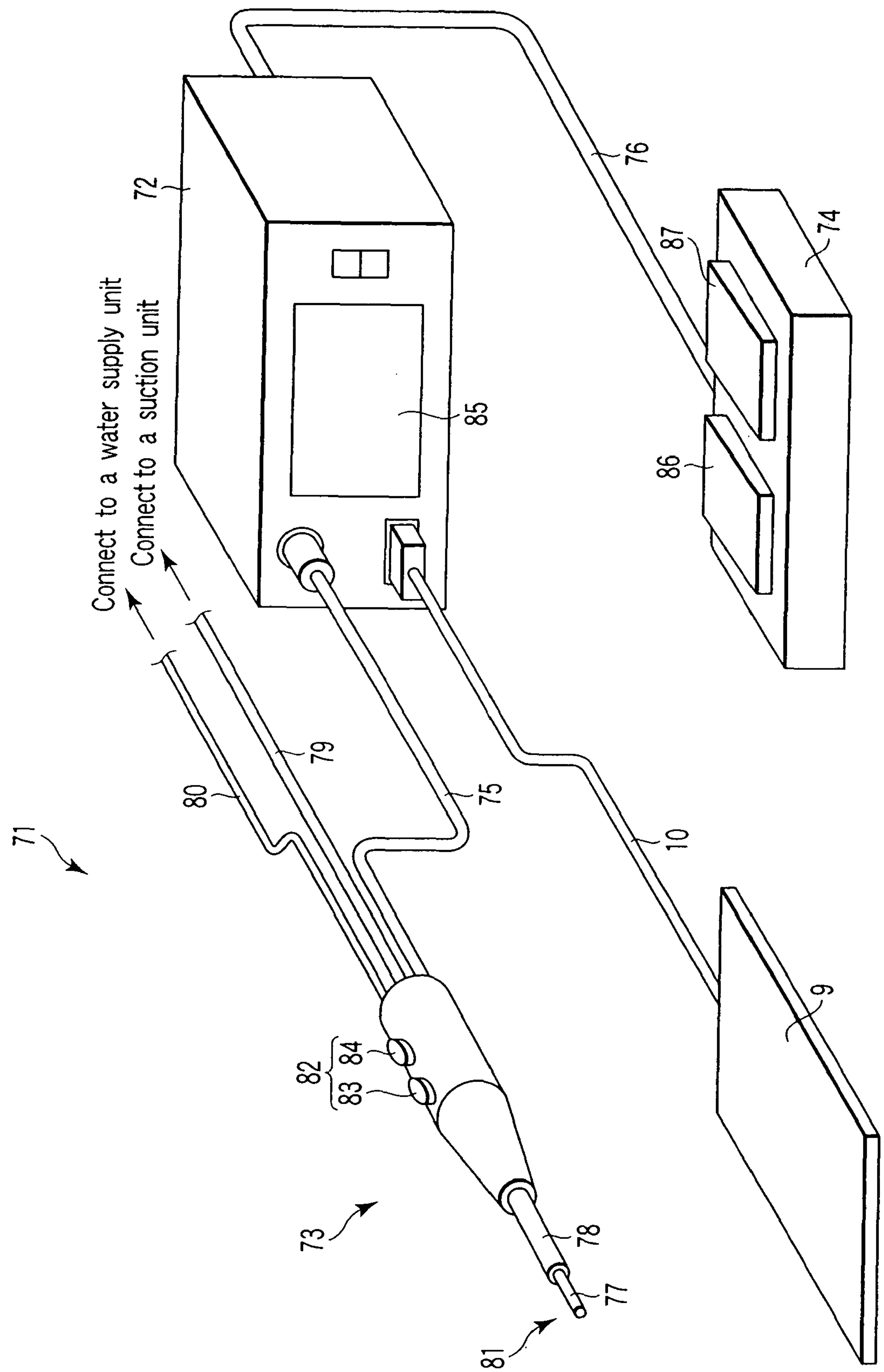
Connect to a water supply unit
Connect to a suction unit
71
72
73
74
75
76
77
78
79
80
81
82
83
84
85
86
87
10
9

FIG. 20

Table of output modes

| High-frequency output | | | Ultrasonic output | Suction | Water supply | No |
|---|---|---|---|---|---|---|
| Monopolar | Cut | Pure | On | Off | Off | 1 |
| | | | Off | Off | Off | 2 |
| | | | Auto | Off | Off | 3 |
| | | Blend | On | Off | Off | 4 |
| | | | Off | Off | Off | 5 |
| | | | Auto | Off | Off | 6 |
| | Coag | Soft | On | Off | Off | 7 |
| | | | Off | Off | Off | 8 |
| | | | Auto | Off | Off | 9 |
| | | Hard | On | Off | Off | 10 |
| | | | Off | Off | Off | 11 |
| | | | Auto | Off | Off | 12 |
| | | Spray | On | Off | Off | 13 |
| | | | Off | Off | Off | 14 |
| | | | Auto | Off | Off | 15 |
| Off | | | On | Auto | Auto | 16 |
| | | | Max | Auto | Auto | 17 |
| Off | | | Off | Low | Off | 18 |
| | | | | High | Off | 19 |
| Off | | | Off | Off | Low | 20 |
| | | | | | High | 21 |

F I G. 21

| Hand switch/Foot switch | | Output mode |
|---|---|---|
| First switch (hand switch) | First output mode | No20 Water supply : Low |
| | Second output mode | No21 Water supply : High |
| Second switch (hand switch) | First output mode | No18 Suction : Low |
| | Second output mode | No19 Suction : High |
| First pedal switch (foot switch) | First output mode | No16 Ultrasonic output : On<br>Suction/water supply : Auto |
| | Second output mode | No17 Ultrasonic output : Max<br>Suction/water supply :Auto |
| Second pedal switch (foot switch) | First output mode | No11 High-frequency output : Monopolar Coag - Hard<br>Ultrasonic output : Off |
| | Second output mode | No12 High-frequency output : Monopolar Coag - Hard<br>Ultrasonic output : Auto |

F I G. 22

European Patent Office

# EUROPEAN SEARCH REPORT

Application Number
EP 08 00 3367

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/040672 A1 (OGURA GEN [JP] ET AL) 27 February 2003 (2003-02-27)<br>* paragraphs [0008] - [0014], [0018] - [0021], [0030] - [0035], [0041] - [0048], [0053] - [0065] *<br>* figures 1,3,5 *<br>* abstract *<br>----- | 1-7 | INV.<br>A61B17/22<br>A61B17/32<br>A61B18/12 |
| X | US 2007/043297 A1 (MIYAZAWA TARO [JP]) 22 February 2007 (2007-02-22)<br>* paragraphs [0034] - [0040], [0088], [0089]; claim 1 *<br>* figures 1,2,12 *<br>* abstract *<br>----- | 1-4,7 | |
| E | EP 1 894 532 A (OLYMPUS MEDICAL SYSTEMS CORP [JP]) 5 March 2008 (2008-03-05)<br>* paragraphs [0022] - [0036], [0051], [0058], [0075] - [0082], [0085] *<br>* figures 1,5-11,14 *<br>* abstract *<br>----- | 1-4,7 | |
| | | | TECHNICAL FIELDS SEARCHED (IPC) |
| | | | A61B |

The present search report has been drawn up for all claims

| Place of search | Date of completion of the search | Examiner |
|---|---|---|
| Munich | 11 June 2008 | Lins, Stephanie |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 08 00 3367

This annex lists the patent family members relating to the patent documents cited in the above-mentioned European search report.
The members are as contained in the European Patent Office EDP file on
The European Patent Office is in no way liable for these particulars which are merely given for the purpose of information.

11-06-2008

| Patent document cited in search report | Publication date | Patent family member(s) | Publication date |
|---|---|---|---|
| US 2003040672 A1 | 27-02-2003 | JP 2003033367 A | 04-02-2003 |
| US 2007043297 A1 | 22-02-2007 | JP 2007050181 A | 01-03-2007 |
| EP 1894532 A | 05-03-2008 | JP 2008055151 A | 13-03-2008 |
| | | US 2008058803 A1 | 06-03-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002306507 A